(19) Europäisches Patentamt
European Patent Office
Office européen des brevets

(11) Veröffentlichungsnummer : **0 337 101 B1**

(12) **EUROPÄISCHE PATENTSCHRIFT**

(45) Veröffentlichungstag der Patentschrift :
**08.07.92 Patentblatt 92/28**

(51) Int. Cl.$^5$ : **C07C 11/02,** C07C 1/20

(21) Anmeldenummer : **89103868.9**

(22) Anmeldetag : **06.03.89**

# (54) Verfahren zur Herstellung von tert.-Olefinen.

(30) Priorität : **15.03.88 DE 3808498**

(43) Veröffentlichungstag der Anmeldung :
**18.10.89 Patentblatt 89/42**

(45) Bekanntmachung des Hinweises auf die Patenterteilung :
**08.07.92 Patentblatt 92/28**

(84) Benannte Vertragsstaaten :
**DE FR GB IT**

(56) Entgegenhaltungen :
**EP-A- 0 102 840**
**APPLIED CATALYSIS, 38, 1988, pages 327-340, Elsevier Science Publishers B.V., Amsterdam**

(73) Patentinhaber : **EC ERDÖLCHEMIE GMBH**
**Postfach 75 20 02**
**W-5000 Köln 71 (DE)**
Patentinhaber : **BAYER AG**
**W-5090 Leverkusen 1 Bayerwerk (DE)**

(72) Erfinder : **Schleppinghoff, Bernhard, Dr.**
**Adolf-Kolping-Strasse 5**
**W-4047 Dormagen 1 (DE)**
Erfinder : **Niederberger, Hans-Ludwig, Dr.**
**Goethestrasse 63**
**W-4047 Dormagen 1 (DE)**
Erfinder : **Gabel, Christian**
**Goethestrasse 69**
**W-4047 Dormagen 1 (DE)**
Erfinder : **Lange, Peter Michael, Dr.**
**Walter-Flex-Strasse 9**
**W-5090 Leverkusen 1 (DE)**
Erfinder : **Mitschker, Alfred, Dr.**
**Am Gartenfeld 50**
**W-5068 Odenthal-Holz (DE)**

(74) Vertreter : **Zobel, Manfred, Dr. et al**
**c/o BAYER AG Konzernverwaltung RP Patente Konzern**
**W-5090 Leverkusen 1 Bayerwerk (DE)**

EP 0 337 101 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von tert.-Olefinen durch Spaltung ihrer Alkylether an speziellen Katalysatoren, die die Bildung der unerwünschten Dialkylether sehr stark unterdrükken und gleichzeitig eine hohe Standzeit aufweisen.

Es ist seit langem bekannt, tert.-Olefine aus Destillatschnitten (z.B. aus Abströmen von Krackern) in reiner Form so zu gewinnen, daß man sie zunächst selektiv mit Alkanolen in ihre Alkylether umwandelt und dann diese Alkylether nach ihrer Abtrennung vom restlichen Destillatschnitt wieder spaltet. Als Spaltkatalysatoren sind saure Kationenaustauscher, z.B. Sulfonsäuregruppen enthaltende Styrol-Divinylbenzol-Harze, neben anderen Katalysatoren bekannt geworden (siche Applied Catalysis, 38 (1988) 327-340 Elsevier Science Publishers BV, Amsterdam).

Alle Spaltkatalysatoren bilden aus dem bei der Spaltung frei werdendem Alkanol den zugehörigen Dialkylether, der stärker flüchtig ist als das zugrundeliegende Alkanol und damit das als Kopfprodukt destillativ abgetrennte tert.-Olefin begleitet und verunreinigt. In besonders hohem Maße gilt dies für den bei der Spaltung von Methyl-tert.-butylether (MTBE) bzw. von tert.-Amylmethylether (TAME) auftretenden Dimethylether, der das gewünschte i-Buten bzw. tert.-Amylen verunreinigt.

Bei Versuchen, die Bildung dieser Dialkylether zurückzudrängen, wurden nur Katalysatoren erhalten, die ihre Altivität und Selektivität bezüglich der Gewinnung reiner tert.-Olefine nach wenigen Tagen, oft nur wenigen Stunden so stark verloren, daß sie für kostengünstige kontinuierliche Verfahren überhaupt nicht in Frage kommen. Es schien also, als ob die sich "öffnende Schere", nämlich die Hinnahme des Nachteils der großen Verunreinigung des tert.-Olefins durch den Dialkylether oder die Hinnahme des Nachteils der unerträglich kurzen Standzeiten des Katalysators nicht zu schließen sei.

Überraschenderweise wurde nun gefunden, daß beide Nachteile gleichzeitig unterdrückt werden können, wenn man als Spaltkatalysatoren die unten beschriebenen einsetzt.

Es wurde also ein Verfahren zur Herstellung von tert.-Olefinen der Formel

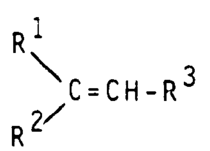 (I)

durch Spaltung ihrer Alkylether der Formel

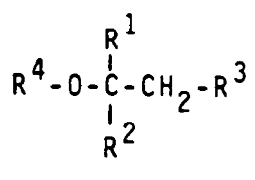 (II) ,

wobei in beiden Formeln

$R^1$, $R^2$, $R^3$ und $R^4$ unabhängig voneinander geradkettiges oder verzweigtes $C_1$-$C_4$-Alkyl bedeuten und $R^3$ zusätzlich Wasserstoff bedeuten kann,

bei erhöhter Temperatur an Sulfonsäuregruppen enthaltenden Styrol-Divinylbenzol-Harzen als Katalysatoren gefunden, das dadurch gekennzeichnet ist, daß man als Katalysatoren solche mit makroporöser Struktur einsetzt, deren Polymermatrix eine innere Oberfläche von 400 - 1000 $m^2/g$, ein Porenvolumen (Porosität) von 0,6 - 2,5 ml/g, bevorzugt 1 - 1,8 ml/g und einen mittleren Porendurchmesser von 40 - 1000 Å, bevorzugt 40 - 300 Å, aufweist und die Spaltung bei 60 - 180°C in der Sumpfphase oder Gasphase durchgeführt wird.

Solche Polymermatrices können nach bekannten Methoden hergestellt und mit bekannten Sulfonierungsmitteln (gasförmiges $SO_3$, Oleum, $H_2SO_4$, $ClSO_3H$ u.a.) mit Sulfonsäuregruppen ausgestattet werden. In bevorzugter Weise wird die Sulfonierung mit 80 - 100 %iger, besonders bevorzugt 85 - 95 %iger $H_2SO_4$ bei 20 - 180°C, bevorzugt 80 - 120°C durchgeführt. Die genannten Herstellungsbedingungen der erfindungsgemäß einzusetzenden Katalysatorharze stellen eine Auswahl unter industriell durchgeführten Verfahren dar und sind damit in einfacher Weise praktikabel und kostengünstig. Die Bedingungen der Sulfonierung führen nicht nur zu einer Sulfonierung im Bereich der Oberfläche, jedoch wird das Polymerpartikel nicht im Kernbereich in gleicher Weise wie an der Ober fläche sulfoniert; eine nennenswerte Sulfonierung wird also in einem äußeren Bereich erreicht, der etwa 1/3 bis 1/2 Partikelradius umfaßt. Hierbei werden Sulfonierungswerte von 1 - 2,5

meq/g, bevorzugt 1,3 - 1,9 meq/g eingestellt.

Das erfindungsgemäße Verfahren wird bei 60 - 180°C in der Gasphase oder in der Sumpfphase mit anschließender destillativer Trennung von tert.-Olefin und Alkanol (vergesellschaftet mit wenig nicht umgesetztem Ether, der recyclisiert wird) durchgeführt. Die Reaktionstemperatur wird vor allem von den Siedepunkten der Reaktionspartner abhängig gemacht. Für dis Gasphase gilt vor allem der obere Bereich von 90 - 180°C, bevorzugt 100 - 170°C, besonders bevorzugt 100 - 160°C. Für die Sumpfphase gilt vor allem der untere Bereich, der allerdings durch Arbeiten unter Druck bis 180°C ausgedehnt werden kann. Beispielsweise werden für die Sumpfphase Temperaturen von 60 - 180°C, bevorzugt 60 - 170°C, besonders bevorzugt 70 - 120°C genannt, wobei man in Abhängigkeit von den Siedepunkten bei einem Druck von 1 - 20 bar, bevorzugt 1 - 10 bar, besonders bevorzugt 1 - 5 bar arbeiten kann. Man kann hierzu bei dem sich bei der gewählten Reaktionstemperatur einstellenden Eigendruck arbeiten oder zusätzlich einen Inertgasdruck anwenden ($N_2$, $H_2$).

Das Arbeiten in der Sumpfphase unter dem sich einstellenden Eigendruck des Systems ist bevorzugt. Hierzu wird bei kontinuierlicher Fahrweise eine Raumgeschwindigkeit LHSV (liquid hourly space velocity) von 1 - 50 l Alkylether pro Stunde und 1 Katalysator, bevorzugt 2 - 20 l/h . l, besonders bevorzugt 2 - 15 l/h . l eingestellt.

Das erfindungsgemäße Verfahren wird in üblichen Reaktionsapparaturen durchgeführt. Für den Fall der Anordnung des Katalysators in einer festen Packung kann den Katalysatorpartikeln Inertmaterial zugemischt werden oder Katalysatorschichten durch Inertschichten unterbrochen werden. Inertmaterialien sind beispielsweise $Al_2O_3$, Stahlkörper, Keramikkörper u.a.

Als zu spaltende Alkylether kommen bevorzugt solche in Frage, bei denen $R^1$ und $R^3$ Methyl bedeuten, wobei $R^3$ zusätzlich Wasserstoff bedeuten kann, und $R^2$ $C_1$-$C_3$-Alkyl, besonders bevorzugt $C_1$-$C_2$-Alkyl bedeutet. Als zu spaltende Alkylether kommen weiterhin bevorzugt solche in Frage, bei denen $R^4$ $C_1$-$C_3$-Alkyl, besonders bevorzugt $C_1$-$C_2$-Alkyl bedeutet.

Die erfindungsgemäß einzusetzenden Katalysatoren haben Standzeiten von mehreren Monaten bei hoher Selektivität der Herstellung von tert.-Olefinen bei sehr geringer Bildung von Dialkylethern. Als Beispiel sei die Bildung des Dimethylethers (DME) aus MTBE bzw. TAME angegeben, die bei herkömmlichen sauren Kationenaustauschern zu einem Gehalt von 0,6 - 1 Gew.-% DME in dem als Kopfstrom abgezogenen i-Buten bzw. i-Amylen führte, während das erfindungsgemäße Verfahren einen Gehalt von unter 1000 ppm, vielfach unter 500 ppm DME ergibt.

Beispiel 1

104 g eines porösen Perlpolymerisats auf Basis Styrol/Divinylbenzol mit einer Oberfläche von 600 $m^2/g$, einem Porenvolumen von 1,6 ml/g und einem mittleren Porendurchmesser von 110 Å wurden gemeinsam mit 500 ml 93 %iger $H_2SO_4$ auf 100°C erwärmt und anschließend 1 h bei dieser Temperatur gerührt. Nach dem Abkühlen der Reaktionsmischung wurde überschüssige $H_2SO_4$ abgehebert und das sulfonierte Harz in Eiswasser eingetragen. Danach wurde in einer Säulenapparatur die restliche $H_2SO_4$ mit vollentsalztem Wasser eluiert. Danach wurde das Harz im Trockenschrank bei 80°C getrocknet. Man erhielt einen Kationenaustauscher, dessen aromatische Kerne zu 27,8 % sulfoniert worden waren; das entspricht 1,8 meq/g.

Beispiel 2

Die Sulfonierung nach Beispiel 1 wurde wiederholt, das Reaktionsgemisch jedoch 6 h bei 120°C gerührt. Hierbei waren die aromatischen Kerne zu 40 % sulfoniert worden.

Beispiel 3

Die Sulfonierung nach Beispiel 1 wurde wiederholt, das Reaktionsgemisch jedoch 6 h bei 180°C gerührt. Hierbei waren die aromatischen Kerne zu 42 % sulfoniert worden; das entspricht 2,56 meq/g.

Beispiel 4

TAME-Spaltung in der Gasphase an einem Katalysator nach Beispiel 1.

Als Alkylether-Spaltreaktor wurde ein temperierbarer Durchlaufreaktor eingesetzt. Bei einem vorgegebenen lichten Reaktordurchmesser von 18 mm wurde die Katalysatorbetthöhe so gewählt, daß die Katalysatorfüllung 150 ml betrug. Zur Temperaturkontrolle war der Reaktor mit mehreren Temperaturmeßstellen ausgerüstet. Der Reaktionsdruck stellte sich, entsprechend dem Druckverlust über der Katalysatorschüttung, von selbst ein. Die Zudosierung des Substrates erfolgte über eine Kolbenpumpe und wurde in einem Vorwärmer auf Reaktionstemperatur erwärmt. Die Zusammensetzung des am Reaktorausgang erhaltenen Reaktionsproduktes wurde gaschromatographisch untersucht.

Der zu spaltende tert.-Amyl-methylether (TAME) hatte folgende Zusammensetzung:

| | |
|---|---|
| TAME | 98 Gew.-% |
| TAA (tert.-Amylalkohol) | 0,9 Gew.-% |
| tert. $C_7$-Ether | 0,5 Gew.-% |
| Methanol | 0,1 Gew.-% |
| Wasser | 0,1 Gew.-% |
| Benzol | 0,2 Gew.-% |
| sonstige Kohlenwasserstoffe | 0,2 Gew.-% |

Unabhängig von den untersuchten Belastungszuständen des Katalysators (Belastung = ml Substrat/ml Kontakt . h) wurden weniger als 500 ppm DME (Dimethylether) im Reaktionsprodukt gefunden. In der Tabelle ist der DME-Gehalt des Reaktionsproduktes für verschiedene Belastungen des Katalysators aufgeführt. Zum Vergleich wurde der DME-Gehalt im Reaktionsprodukt aus der TAME- Spaltung an einem handelsüblichen Ionenaustauscherharz (SPC 118 der Fa. Bayer AG) mit in die Tabelle aufgenommen. Die Reaktionstemperatur lag zwischen 120 und 150°C.

| Katalysator | LHSV [ml/ml.h] | DME-Gehalt Massen-ppm |
|---|---|---|
| Katalysator nach Beispiel 1 | 14,7 | <100 |
| | 8,7 | 140 |
| | 5,0 | 320 |
| SPC 118 | 7 | 3000 |

Die Standzeit des Katalysators nach Beispiel 1 ist außerordentlich hoch. Während einer Versuchslaufzeit von über 600 Stunden konnte keine Desaktivierung festgestellt werden.

Beispiel 5 (Vergleichsbeispiel)

MTBE-Spaltung und gleichzeitige Methanolextraktion in einer Kolonne:

In der im folgenden beschriebenen Spaltkolonne wurde über 90 Tage MTBE zu i-Buten und Methanol gespalten:

Eine mit Destillations- und Extraktionseinrichtungen versehene Druckkolonne aus Stahl mit einem Innendurchmesser von 50 mm wurde im Sumpfumlauf mit einer Schüttung von Spaltkontakt ausgerüstet. Zum Sumpfumlauf gehörte ferner die indirekte Sumpfheizung. Auf den Verdampfer waren drei Kolonnenschüsse zu je 1 Meter montiert. Die Kolonne hatte 20 praktische Böden und war für einen Betriebsdruck bis zu 20 bar ausgelegt. Am Kopf wurde der Kolonne Wasser zugeführt, das in der Mitte wieder entnommen wird; dieses Wasser enthielt den überwiegenden Teil des bei der Spaltung entstehenden Methanols. Der zu spaltende MTBE wurde über den Sumpf der Kolonne zugeführt. Am Kolonnenkopf wurde das bei der Spaltung entstehende i-Buten entnommen.

Reaktionsbedingungen:

| | |
|---|---|
| Einsatz MTBE (gaschromatographisch) | 99,9 Gew.-% |
| Verhältnis Waschwasser zu MTBE | 1 : 1 |
| Sumpftemperatur (Spalttemperatur) | 94°C |
| Kopftemperatur | 39,5°C |
| Kolonnendruck | 3,5 bar |
| Rücklaufverhältnis | 6,0 |
| Katalysatormenge | 0,5 l |
| LHSV [ml/ml.h] | 8,0 |

Als Spaltkatalysator wurde starksaurer, makroporöser handelsüblicher SPC 118 von Bayer eingesetzt.

Das als Kopfprodukt anfallende i-Buten wurde gaschromatographisch analysiert und hatte folgende Zusammensetzung:

| | |
|---|---|
| i-Buten | 99,4 Gew.-% |
| Dimethylether | 0,58 Gew.-% |
| $H_2O$ | 0,02 Gew.-% |

Diese Zusammensetzung war während der gesamten Laufzeit konstant.

Beispiel 6

Der Versuch wurde wie der in Beispiel 5 beschriebene durchgeführt, als Spaltkontakt wurde der nach Beispiel 1 eingesetzt.

Die Zusammensetzung des hierbei anfallenden Kopfproduktes war folgendermaßen:

| | |
|---|---|
| i-Buten | 99,9 Gew.-% |
| Dimethylether | 0,03 Gew.-% |
| Wasser | 0,02 Gew.-% |

Während des gesamten Untersuchungszeitraumes von 90 Tagen blieb diese Zusammensetzung konstant, ein Abklingen der Katalysatoraktivität war nicht zu erkennen.

## Patentansprüche

1. Verfahren zur Herstellung von tert.-Olefinen der Formel

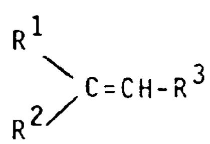

durch Spaltung ihrer Alkylether der Formel

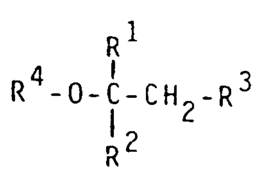

,

wobei in beiden Formeln

$R^1$ , $R^2$, $R^3$ und $R^4$ unabhängig voneinander geradkettiges oder verzweigtes $C_1$-$C_4$-Alkyl bedeuten und $R^3$ zusätzlich Wasserstoff bedeuten kann,
bei erhöhter Temperatur an Sulfonsäuregruppen enthaltenden Styrol-Divinylbenzol-Harzen als Katalysatoren, dadurch gekennzeichnet, daß man als Katalysatoren solche mit makroporöser Struktur einsetzt, deren Polymermatrix eine innere Oberfläche von 400 - 1000 $m^2/g$, ein Porenvolumen (porosität) von 0,6 - 2,5 ml/g und einen mittleren Porendurchmesser von 40 - 1000 Å aufweist und die Spaltung bei 60 - 180°C in der Sumpfphase oder Gasphase durchgeführt wird.

2. Verfahren nach Anspruch 1, dadurch gekennzeichnet, daß der mittlere Porendurchmesser 40 - 300 Å beträgt.

3. Verfahren nach Anspruch 1, dadurch gekennzeichnet, daß das Porenvolumen (Porosität) 1 - 1,8 ml/g beträgt.

4. Verfahren nach Anspruch 1, dadurch gekennzeichnet, daß die Sulfonsäuregruppen durch Behandlung der Matrix mit 80 - 100 %iger $H_2SO_4$, bevorzugt mit 85 - 95 %iger $H_2SO_4$ bei 20 - 180°C, bevorzugt 80 - 120°C eingeführt werden.

5. Verfahren nach Anspruch 1, dadurch gekennzeichnet, daß die Spaltung bei 60 - 180°C, bevorzugt 60 - 170°C, besonders bevorzugt 70 - 120°C und einem Druck von 1 - 20 bar, bevorzugt 1 - 10 bar, besonders bevorzugt 1 - 5 bar in der Sumpfphase durchgeführt wird.

6. Verfahren nach Anspruch 5, dadurch gekennzeichnet, daß eine Raumgeschwindigkeit LHSV (liquid hourly space velocity) von 1 - 50 l Alkylether pro Stunde pro 1 Katalysator, bevorzugt 2 - 20 l/h.l, besonders bevorzugt 2 - 15 l/h.l eingestellt wird.

7. Verfahren nach Anspruch 1, dadurch gekennzeichnet, daß $R^1$ und $R^3$ Methyl bedeuten, wobei $R^3$ zusätzlich Wasserstoff bedeuten kann, und $R^2$ $C_1$-$C_3$-Alkyl, bevorzugt $C_1$-$C_2$-Alkyl bedeutet.

8. Verfahren nach Anspruch 1, dadurch gekennzeichnet, daß $R^4$ $C_1$-$C_3$-Alkyl, bevorzugt $C_1$-$C_2$-Alkyl bedeutet.

## Claims

1. Process for the preparation of tert.-olefins of the formula

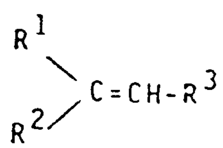

by cleavage of their alkyl ethers of the formula

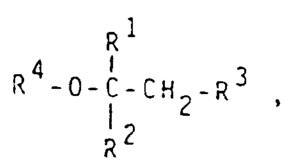

where, in the two formulae,
$R^1$, $R^2$, $R^3$ and $R^4$ independently of one another denote straight-chain or branched $C_1$-$C_4$-alkyl and $R^3$ can additionally denote hydrogen,
at an elevated temperature over styrene/divinylbenzene resins, containing sulphonic acid groups, as catalysts, characterized in that the catalysts used are those which have a macroporous structure and the polymer matrix of which has an internal surface area of 400 - 1000 $m^2/g$, a pore volume (porosity) of 0.6 - 2.5 ml/g and a mean pore diameter of 40 - 1000 Å, and the cleavage is carried out at 60 - 180°C in the liquid phase or gas phase.

2. Process according to Claim 1, characterized in that the mean pore diameter is 40 - 300 Å.

3. Process according to Claim 1, characterized in that the pore volume (porosity) is 1 - 1.8 ml/g.

4. Process according to Claim 1, characterized in that the sulphonic acid groups are introduced by treating the matrix with 80 - 100 % strength $H_2SO_4$, preferably with 85 - 95 % strength $H_2SO_4$ at 20 - 180°C, preferably 80 - 120°C.

5. Process according to Claim 1, characterized in that the cleavage is carried out in the liquid phase at 60 - 180°C, preferably 60 - 170°C, particularly preferably 70 - 120°C, and under a pressure of 1 - 20 bar, preferably 1 - 10 bar, particularly preferably 1 - 5 bar.

6. Process according to Claim 5, characterized in that the liquid hourly space velocity (LHSV) is adjusted to 1 - 50 l of alkyl ether per hour per litre of catalyst, preferably 2 - 20 l/hour x litre, particularly preferably 2 - 15 l/hour x litre.

7. Process according to Claim 1, characterized in that $R^1$ and $R^3$ denote methyl, it being possible for $R^3$ additionally to denote hydrogen, and $R^2$ denotes $C_1$-$C_3$-alkyl, preferably $C_1$-$C_2$-alkyl.

8. Process according to Claim 1, characterized in that $R^4$ denotes $C_1$-$C_3$-alkyl, preferably $C_1$-$C_2$-alkyl.

## Revendications

1. Procédé de préparation de tert-oléfines de formule

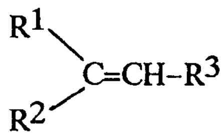

par scission de leurs éthers alkyliques de formule

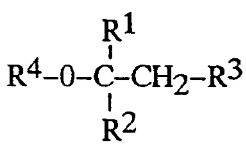 ,

les symboles des deux formules ayant les significations suivantes:
$R^1$, $R^2$, $R^3$ et $R^4$ représentent chacun, indépendamment les uns des autres, un groupe alkyle à chaîne droite ou ramifiée en $C_1$-$C_4$, $R^3$ pouvant en outre représenter l'hydrogène, à température élevée sur des catalyseurs consistant en résines styrène-divinylbenzène portant des groupes acide sulfonique,
ce procédé se caractérisant en ce que l'on utilise en tant que catalyseur de telles résines à structure macroporeuse, dont la gangue polymère a une surface interne de 400 à 1 000 $m^2$/g, un volume de pores (porosité) de 0,6 à 2,5 ml/g et un diamètre de pore moyen de 40 à 1 000 Å et en ce que l'on procède à la scission à des températures de 60 à 180°C en phase pied de colonne ou en phase gazeuse.

2. Procédé selon la revendication 1, caractérisé en ce que le diamètre de pore moyen est de 40 à 300 Å.

3. Procédé selon la revendication 1, caractérisé en ce que le volume de pores (porosité) est de 1 à 1,8 ml/g.

4. Procédé selon la revendication 1, caractérisé en ce que les groupes acide sulfonique sont introduits par traitement de la gangue à l'aide de l'acide sulfurique à une concentration de 80 à 100 %, de préférence de 85 à 95 %, à des températures de 20 à 180°C, de préférence de 80 à 120°C.

5. Procédé selon la revendication 1, caractérisé en ce que la scission est réalisée à des températures de 60 à 180°C, de préférence de 60 à 170°C, plus spécialement de 70 à 120°C et à une pression de 1 à 20 bar, de préférence de 1 à 10 bar, plus spécialement de 1 à 5 bar, en phase pied de colonne.

6. Procédé selon la revendication 5, caractérisé en ce que l'on règle à une vitesse spatiale horaire de liquide VSHL de 1 à 50 l d'éther alkylique par heure et par litre de catalyseur, de préférence de 2 à 20 l/h.l et plus spécialement de 2 à 15 l/h.l.

7. Procédé selon la revendication 1, caractérisé en ce que $R^1$ et $R^3$ représentent des groupes méthyle, $R^3$ pouvant en outre représenter l'hydrogène, et $R^2$ représente un groupe alkyle en $C_1$-$C_3$, de préférence en $C_1$-$C_2$.

8. Procédé selon la revendication 1, caractérisé en ce que $R^4$ représente un groupe alkyle en $C_1$-$C_3$, de préférence en $C_1$-$C_2$.